# EUROPEAN PATENT SPECIFICATION

(11) **EP 4 285 850 B1**
(45) Date of publication and mention of the grant of the patent: **19.08.2026**
(21) Application number: 23175980.4
(22) Date of filing: 30.05.2023
(51) Int. Cl.: A61B 18/14, A61B 5/00

(54) **CATHETER END EFFECTOR PROVIDING MAPPING GRID WITH HIGH DENSITY ELECTRODE ARRAY AND STRAIN REDUCTION**
ENDEFFEKTOR FÜR EINEN KATHETER, UMFASSEND EIN MAPPING-GITTER MIT HOCHDICHTER ELEKTRODENANORDNUNG UND REDUKTION MECHANISCHER SPANNUNG
EFFECTEUR D'EXTRÉMITÉ DE CATHÉTER FOURNISSANT UNE GRILLE DE CARTOGRAPHIE AVEC UN RÉSEAU D'ÉLECTRODES À HAUTE DENSITÉ ET RÉDUCTION DE PRESSION

(30) Priority: 31.05.2022 US 202263347381 P; 21.12.2022 US 202263434273 P; 19.04.2023 US 202318303404
(43) Date of publication of application: 06.12.2023
(73) Proprietor: BIOSENSE WEBSTER (ISRAEL) LTD., 2066717 Yokneam (IL)
(72) Inventor: VAN NIEKERK, Pieter Emmelius, Irvine, 92618 (US); TOBEY, Dustin R., Irvine, 92618 (US); PATEL, Amar, Irvine, 92618 (US); EBRAHIMI, Babak, Irvine, 92618 (US); KNUDSON, John Christian, Irvine, 92618 (US)
(74) Representative: Carpmaels & Ransford LLP

(56) References cited:
- EP-A1- 3 915 477
- US-A1- 2012 271 138
- US-A1- 2020 345 262
- US-A1- 2021 015 551
- US-A1- 2021 369 339
- US-A1- 2022 054 198

## Description

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Important sources of undesired signals are located in the tissue region, for example, one of the atria or one of the ventricles. Regardless of the sources, unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure--mapping followed by ablation--electrical activity at points in the heart is typically sensed and measured by advancing a catheter containing one or more electrical sensors into the heart and acquiring data at a multiplicity of points. These data are then utilized to select the target areas at which ablation is to be performed.

For greater mapping resolution, it is desirable for a mapping catheter to provide very high-density signal maps through the use of a multitude of electrodes sensing electrical activity within a small area, for example, a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature.
US 2012/271138 A1 discusses a system for sensing multiple local electric voltages from endocardial surface of a heart, including: an elongate tubular member; a plurality of flexible splines having proximal portions, distal portions and medial portions therein between; an anchor for securably affixing the proximal portions of the splines; a tip for securably affixing the distal portions of the splines; and a polymeric member including opposed a first open end and a second open end defining an open lumen therein between and an inner member surface and an outer member surface, wherein at least one of the plurality of flexible splines is at least partially disposed within the lumen of the polymeric member; a flexible electrode assembly strip with one or more exposed electrodes disposed on at least a portion of the outer surface of the polymeric member.
EP 3915477 A1 describes an apparatus including an end effector having loop members with electrodes thereon and which is usable with catheter-based systems to measure or provide electrical signals. The end effector can include three loop members that are non-coplanar when expanded unconstrained that become contiguous to a planar surface when the loop members are deflected against the surface, a mechanical linkage that joins the loop members at a distal vertex of the end effector, electrodes having surface treatment to enhance surface roughness of the electrodes, twisted pair electrode wires, a bonded spine cover, and/or any combination thereof.

### SUMMARY

Various embodiments described herein allow high density mapping and/or ablation of tissue surface in the heart, including an atrium or a ventricle, by virtue of a catheter for electrophysiology applications. The catheter includes a tubular member and an end effector. The tubular member extends along a longitudinal axis from a proximal portion to a distal portion. The end effector is coupled to the distal portion. The end effector of the invention is defined in independent claim 1.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
FIG. 1A illustrates a catheter from an effector at a distal portion of the catheter to the proximal handle;
FIG. 1B is a close up of the distal portion of the end effector;
FIG. 1C is a perspective cut away view of the proximal portion of the end effector;
FIG. 1D is yet another closer view of FIG. 1C with certain components hidden;
FIG. 1E is a cross-sectional view of the distal end of FIG. 1A;
FIG. 2 illustrates an exploded plan view of the underlying frameworks for the end effector of FIG. 1A;
FIG. 3 illustrates a front elevation view of another example of an end effector for use with the catheter of FIG. 1A;
FIG. 3A is a close up of the distal portion of the end effector of FIG. 3, showing stress distribution nodes of the end effector; and
FIG. 3B is a close up of the distal portion of the end effector of FIG. 3, showing a cover positioned over the spines of the end effector.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. The term "proximal" and "distal" are used to reference location of various components with respect to the handle which is designated as the most proximal to a user operating the handle.

### I. Example of a Catheter End Effector

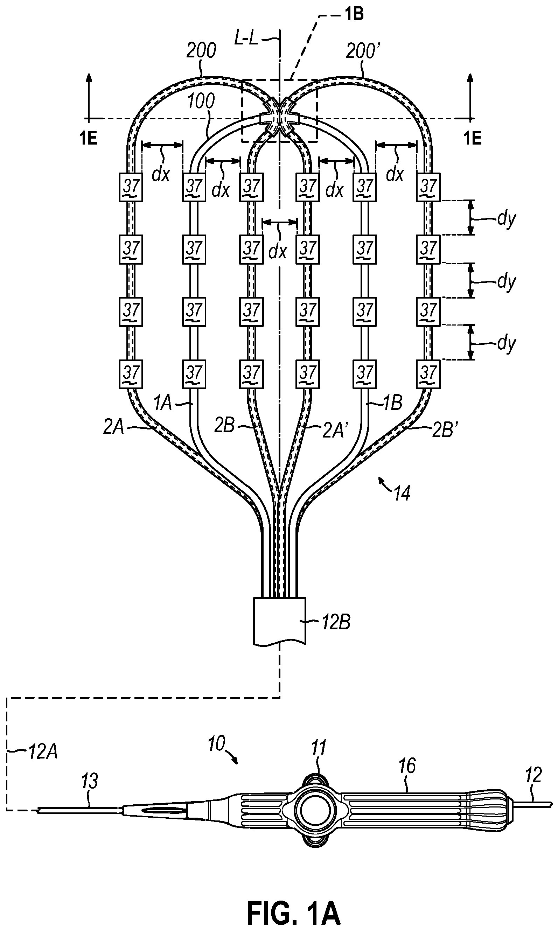

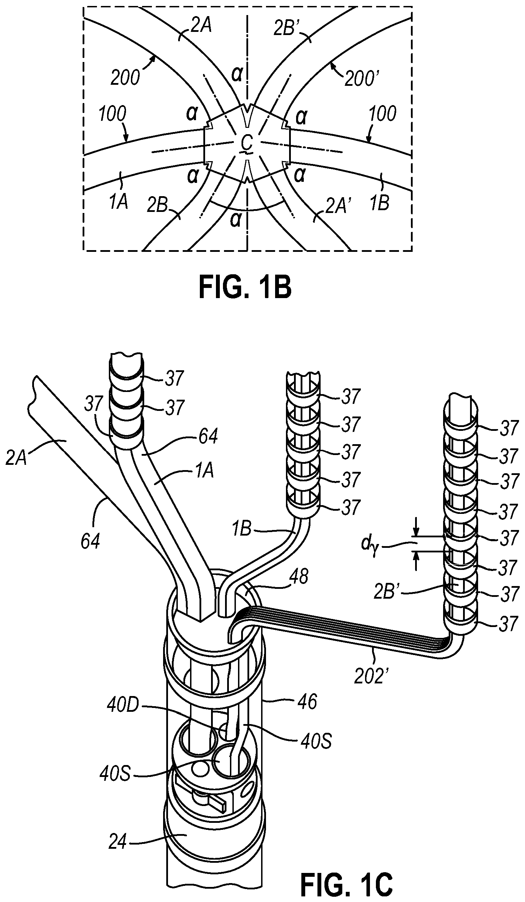

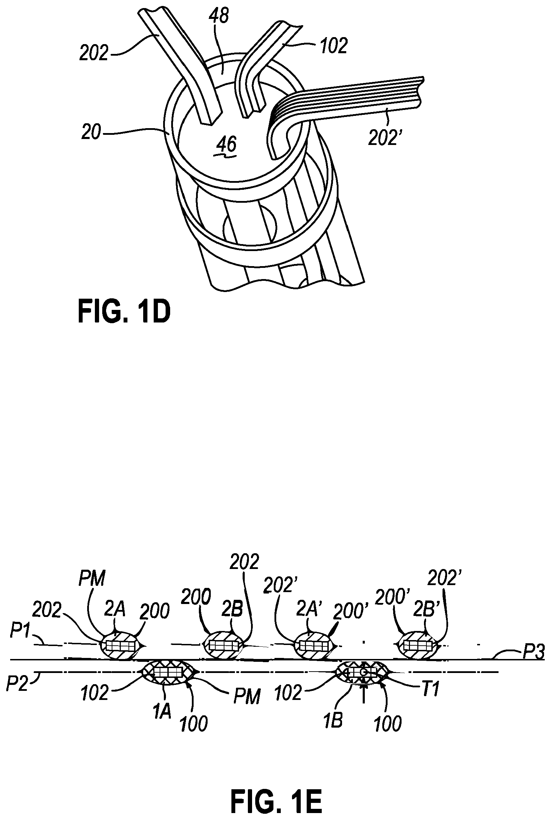

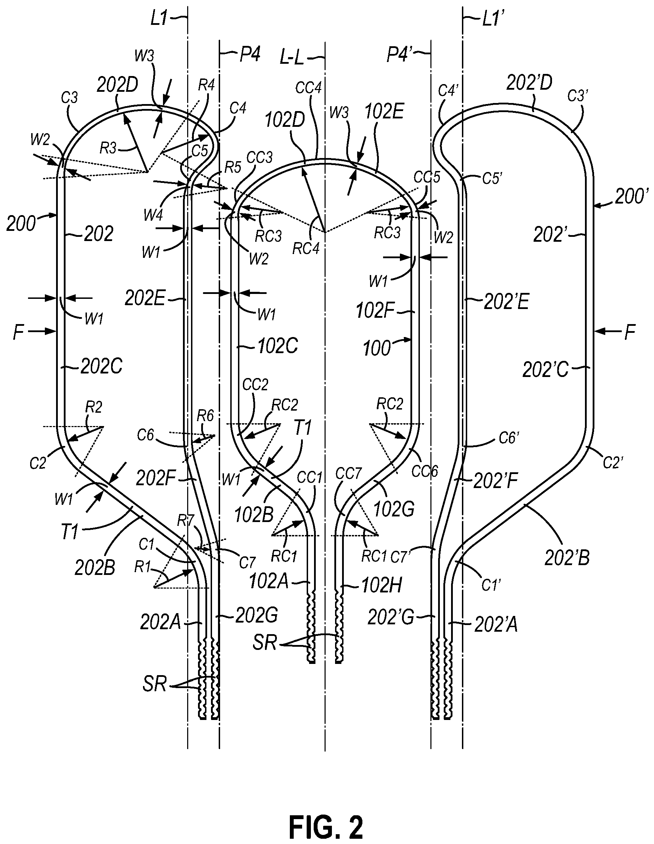

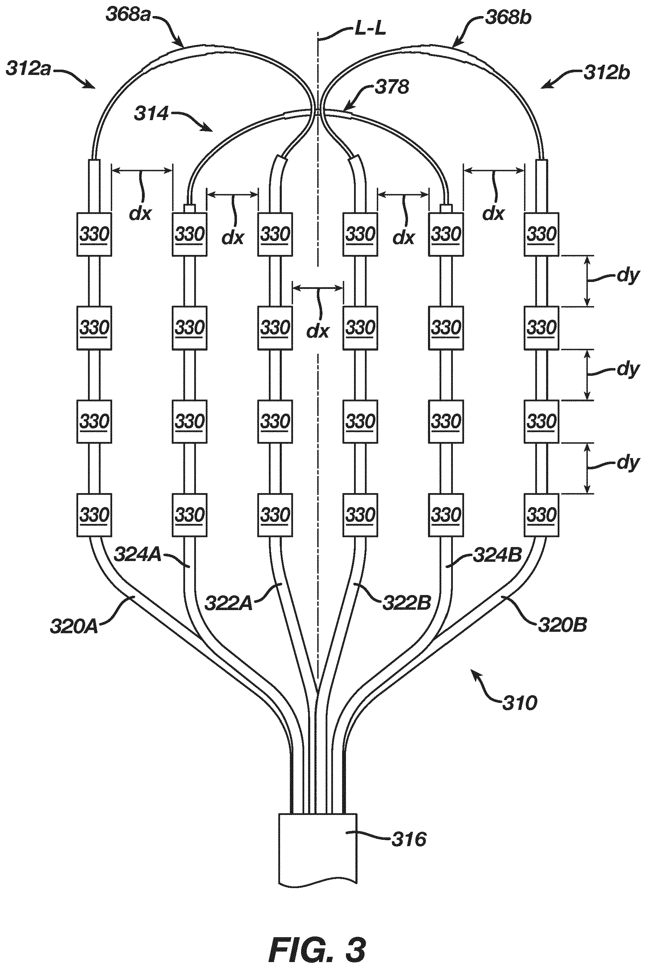

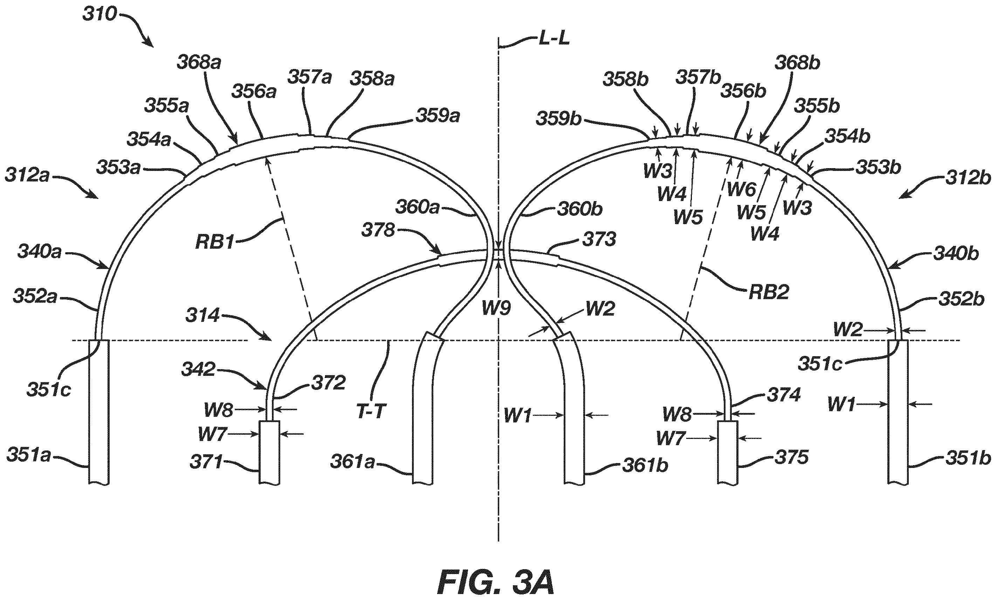

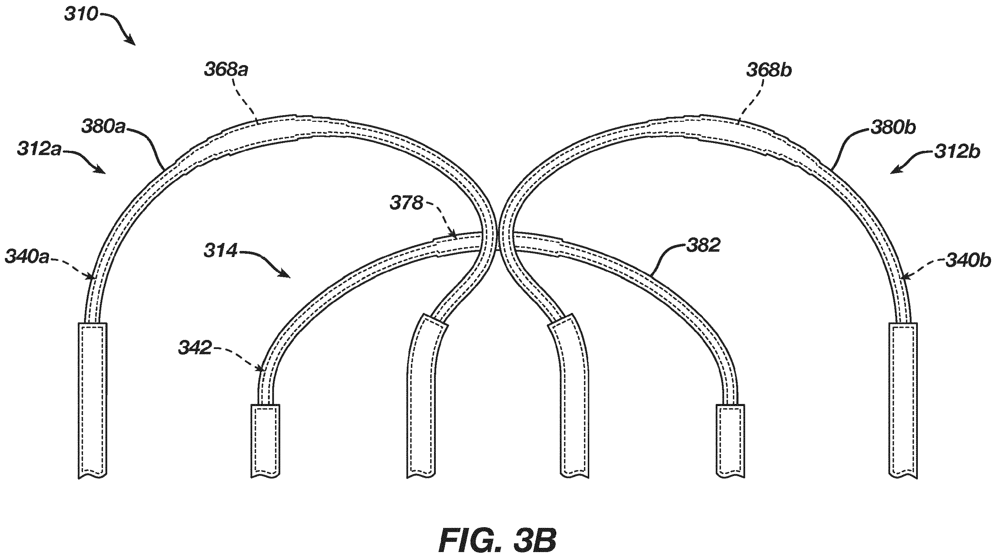

As shown in FIG. 1, the catheter 10 comprises an elongated catheter body 12 disposed inside a separate intermediate tubular section 13, a distal electrode assembly or end effector 14, and a deflection control handle 16 attached to the proximal end of the catheter body 12. In accordance with a feature of the present invention, the effector 14 has a plurality of spines 1A, 1B, 2A, 2B, 3A, 3B that lies on different planes. The intermediate section 13 can extend along a longitudinal axis L-L along a proximal portion 12A to a distal portion 12B before deployment of the end effector 14. One or more impedance sensing electrode can be provided on distal portion 12B (not shown) to allow for location sensing via impedance location sensing technique, as described in U.S. Pat. No. 5,944,022, entitled "Catheter Positioning System," issued August 31, 1999; U.S. Pat. No. 5,983,126, entitled "Catheter Location System and Method," issued November 9, 1999; and U.S. Pat. No. 6,445,864, entitled "Dispersion Compensating Optical Fiber," issued September 3, 2002. Details of the catheter body 16 can be understood from a review of U.S. Pub. No. 2020/0345262, entitled "Mapping Grid with High Density Electrode Array," published November 5, 2020, including the description to the handle and particularly FIGs. 2D, 2E and 3 in this prior application.

Distal portion (12B) is coupled to an end effector (14) in FIG. 1A. The end effector (14) has three loop members (100), (200) and (200') with two loop members (200) and (200') is identical and disposed on either side of central loop member (100). In FIG. 1A, it can be seen that loop (200) is identical to loop (200') except that loop (200') is oriented 180 degrees compared to loop (200) with respect to axis (L-L). That is, loop (200) and loop (200') can be considered to be mirror images of each other. Loop (100) on the other hand is distinct from loops (200) and (200'). The three loops (100, 200 200') are tied together at the distal end shown in FIG. 1B. In FIG. 1B, spine member (1A) is integral with spine member (1B) as part of loop (100) and spine members (2A, 2B) are integral to loop (200). Similarly, spine members (2A', 2B') are integral to loop (200'). These spine members (1A, 1B, 2A, 2B, 2A', 2B') cross at a common center (C) such that each spine member (1A, 1B, 2A, 2B, 2A', 2B') is angled relative to each of its adjacent neighboring spines (1A, 1B, 2A, 2B, 2A', 2B') at a separation angle (α) of approximately 30 degrees. For example, spine (1A) is separated from each of its neighboring spines (2A, 2B) by a respective angle (α).

As shown in FIGs. 1C and 1D, four spines including spines (1A, 1B) of loop (100) and spines (2A, 2B) of loop (200) are shown with the cover (typically polymer such as polyurethane) on spines (1B, 2A, 2B) removed so that structural member (102, 202, 202') for central loop 100 and outer loops (200, 200') can be seen. That is, each outer loop (200, 200') with spine frame (202, 202') and central loop (100) with frame (102) has a respective elongated structural member (102, 202, 202') extending through the length of the loop (100, 200, 200'), to and from insert member (20) which is part of tubular portion (46). While the preferred embodiment of the loops is formed from a single unitary material, it is possible that each segment of each loop (as described in FIG. 2) can be discrete components affixed to each other. A proximal portion of each loop structural member (102, 202, 202') extends into a distal end portion of the connector tubing (46) (which is part of tubular member (12)) and is anchored in the lumen (48) into insert (20). To ensure that the loops (100, 200, 200') can be compressed into a very small shape for delivery into a vessel, we have devised the outer loops (200, 200') to have a structural member (202, 202') in the asymmetric configuration shown in FIG. 2 while structural member (102) for the central loop (100) has the symmetric configuration shown in FIG. 2.

Referring to FIG. 2, structural member (102) for central loop (100) is a unitary structural framework that has been physically manipulated to define different segments. In FIG. 2, it is noted that the central loop (100) includes a continuous central frame (102), in which the continuous central frame (102) may include the first to eighth linear segments (102A, 102B, 102C, 102D, 102E, 102F, 102G, 102H), respectively, with first to seventh curved segments (CC1, CC2, CC3, CC4, CC5, CC6, CC7) disposed between the respective first to eighth linear segments (102A, 102B, 102C, 102D, 102E, 102F, 102G, 102H).

Each of the outer loop members (200, 200') includes a respective first linear segment (202A, 202'A) connected to a respective second linear segment (202B, 202'B) with a respective first curved segment (C1, C1'), the second linear segment (202B, 202'B) is connected to a respective third linear segment (202C, 202'C) via a respective second curved segment (C2, C2'). The third linear segment (202C, 202'C) is connected to a respective distal curved segment (202D, 202'D) which is connected to a respective fourth linear segment (202E, 202'E) which is connected to a respective fifth linear segment (202F, 202'F) via a respective sixth curved segment (C6, C6'), the fifth linear segment (202F, 202'F) is connected to a respective sixth linear segment (202G, 202'G) via a respective seventh curved segment (C7, C7'). The seventh curved segment (C7, C7') is becomes the respective final sixth linear segment (202G, 202'G).

In FIG. 2, it can be seen that distal curved segment (202D, 202'D) of each outer loop (200, 200') may include a respective third curved segment (C3, C3') extending to a respective fourth curved segment (C4, C4') which becomes a respective fifth curved segment (C5, C5') which continues on as the respective fourth linear segment (202E, 202'E). As noted, each of the outer loops (200, 200') can be from a respective continuous outer frame (202, 202') that can be formed from multiple pieces but are preferably unitary.

Of note here is that fourth curved segments (C4, C4') for outer frames (202, 202') are each configured to protrude towards the longitudinal axis L-L in which the respective fourth curved segment (C4, C4') extends beyond a respective virtual center line (L1, L1') of the corresponding fourth linear segment (202E, 202'E) and contiguous (or tangential) to a respective plane (P4, P4') which is parallel to the corresponding sixth linear segment (202G, 202'G) and the longitudinal axis (L-L). This configuration of the outer loops (200, 200') is believed to ensure sufficient structural robustness to allow each of the outer loops (202, 202') to maintain its intended shape when deployed into a much larger planar configuration despite is compressed into a tubular sheath (13) during storage prior to is used in an electrophysiology procedure.

By virtue of this design, the outer frames (202, 202') are disposed on each side of the central loop frame (102) so that one outer loop frame (202) can be seen as a mirror image of the other outer loop frame (202'). As shown in FIG. 1E, a first virtual plane (P1) extends through centers of the outer loops (200, 200') and a second plane (P2) parallel to the first plane (P1) extends through the centers of the central loop (100). Note that the outer loops (200, 200') and the central loop (100) are contiguous to a third plane (P3) disposed between first and second planes (P1, P2).

The central and outer frames (102, 202, 202') each have a generally rectangular cross section of varying dimensions with a maximum width of about 0.25 mm and a minimum width of about 0.1mm with a thickness of about 0.1mm. In particular, the width (W1) of the central frame (102) and outer frames (202, 202') is approximately 0.2mm to 0.3mm for a cross-sectional area of approximately 0.02 mm-squared to 0.03 mm-squared. Width (W2) of each frame (102, 202, 202') is a tapering width (W2) that tapers continuously from the width (W1) to about 0.1 mm for width (W3) for a tapering cross-sectional area starting from 0.03 mm-squared to 0.01 mm-squared. Width (W3) is approximately 0.1 mm for a cross-sectional area of about 0.01 mm-squared. Width (W4) for the outer frames (202, 202') is an increasing width that increases from width (W3) (about 0.1 mm) to a width of any value in the range of approximately 0.2mm to approximately 0.3mm for increasing cross-sectional areas from 0.01 mm-squared to 0.03 mm-squared. Viewing the width of the distal end curves (202D, 202'D) another way, it can be seen that the width (W3) is approximately half of the width (W1) for the main portions (202C, 202E, 202'C, 202'E) of the respective loop frame (202, 202'). At its maximum, width (W4) is now the same as width (W1) for the remainder of the segments all the way to the respective end segment (202G, 202'G). End segments (202G, 202'G) can each be provided with serrations (SR) to allow the end segments (202G, 202'G) to be locked to each other or overmolded into tubular portion (46). The frames (102, 202 and 202') each have the same thickness (T1) (FIG. 1E) of approximately 0.1mm. The frames (102, 202 and 202') can each include a biocompatible metal such as stainless steel, cobalt chromium or nitinol.

In FIG. 2, radius of curvature (R1) and/or radius of curvature (R2) may be about 2mm; radius of curvature (R3) may be about 3mm; radius of curvature (R4) and/or radius of curvature (R5) may be about 1.5 mm; radius of curvature (R6) and/or radius of curvature (R7) maybe about 3mm; radius of curvature (RC1) and/or radius of curvature (RC2) may be about 3mm; radius of curvature (RC3) may be about 1.5 mm; and/or radius of curvature (RC4) may be about 4mm.

While the frames (102, 202, 202') are described with rectangular cross-sections, it should be noted that various non-rectangular (i.e., circular, ovoid, or semi-circular cross-sections) can be used as long as the cross-sections have the same cross-sectional areas as noted above and the described cross-sectional areas are possible.

As shown in FIG. 1A, the completed end effector (14) has a cover in the form of a polymer material (PM) disposed over each of the continuous central and outer frames (102, 202, 202') with a plurality of electrodes (37) disposed on an outer surface of the polymer material (PM). In FIG. 1B, it can be seen that the central loop (100) and the outer loops (200, 200') are physically connected at a distal location (C) of the central frame (102) proximate the longitudinal axis (L-L) such that each of the distal portions (1A, 1B, 2A, 2B, 2A', 2B') are subtended to its neighboring loops by separation angle (α) of approximately 30 degrees. In particular, distal portion (1A) of central loop (100) is separated by its neighboring distal portion (2A) of outer loop (200) and distal portion (2B) of outer loop (200) with approximately the same angle (α). Similarly, distal portion (1B) of central loop (100) is separated by angle (α) from its neighboring distal portion (2A') of outer loop (200') and distal portion (2B') of outer loop (200') by approximately the same angle (α). The physical connection (C) can be in the form of a suture or reflowing of the polymer material PM so that all the members are physically joined together due to the heating and reflowing of the polymer.

Referring back to FIG. 1A, it can be seen that the plurality of electrodes (37) are disposed over the outer loops (200, 200') and the central loop (100) so that the electrodes (37) are spaced apart over a distance from its adjacent neighboring electrodes (37) (as measured from center of one electrode (37) to center of another electrode (37)) by a first distance (dy) along the longitudinal axis (L-L) and a second distance (dx) along an axis transverse to the longitudinal axis (L-L). While the distances (dx, dy) can be different or unequal from each other, the preference is to configure the spacings (dx, dy) so that the first and second distances (dy, dx) are approximately equal to each other.

### II. Example of Alternative Loop Configuration for a Catheter End Effector

In some procedures, it may be desirable to selectively house end effector (14) within tubular sheath (13) and selectively deploy end effector (14) from tubular sheath (13), such that loops (100, 200, 200') are elastically compressed to a collapsed state (not shown) when end effector (14) is housed within tubular sheath (13), and such that loops (100, 200, 200') resiliently expand from the collapsed state to an expanded state in which loops (100, 200, 200') assume the configurations shown in FIG. 1A when end effector (14) is deployed from tubular sheath (13). In addition, or alternatively, it may be desirable to direct end effector (14) into tubular sheath (13) using an insertion tool (not shown). Therefore, it may be desirable to configure loops (100, 200, 200') to distribute stress across loops (100, 200, 200') in a predetermined manner that allows loops (100, 200, 200') to maintain their elasticity and avoid yielding to plastic deformation, which may otherwise occur when the outermost spines (2A, 2B') and/or distal segments (102D, 102E, 202D, 202'D) of loops (100, 200, 200') are subjected to various stress concentrations during repeated cycles of deployment and housing of end effector (14) relative to tubular sheath (13) and/or repeated cycles of being directed into tubular sheath (13) via the insertion tool.

In this regard, loops (100, 200, 200') may each experience two deformation modes upon repeated cycles of deployment and housing: in-plane deformation (also referred to as planar deformation) and out-of-plane deformation. Some cases of planar deformation may include inward bowing of the two outermost spines (2A, 2B') toward each other such that the two outermost spines (2A, 2B') collectively define an hourglass shape, which may occur when the two outermost spines (2A, 2B') experience substantially high stress concentrations at or near their respective middle portions upon entry into tubular sheath (13). Such deformation may be referred to as "hourglass deformation." In addition, or alternatively, some cases of planar deformation may include creasing of any one or more of the distal segments (102D, 102E, 202D, 202'D). Such deformation may be referred to as "distal curve creasing." Some cases of out-of-plane deformation may include inward folding of the two outer loops (200, 200') toward each other around the central loop (100), such as due to the two outermost spines (2A, 2B') is parts of two separate loops (200, 200') which can move independently of each other, deform, and rotate to curl in the direction of collapse. Such deformation may be referred to as "paddle folding."

Distributing stress across loops (100, 200, 200') in a predetermined manner that minimizes or eliminates undesirable stress concentrations may inhibit the aforementioned modes of deformation from occurring, such that loops (100, 200, 200') may consistently and reliably expand from the collapsed state to the desired expanded state in which electrodes (37) may be spaced apart from each other by predefined distances, such as distances (dx, dy) described above. It will be appreciated that avoiding inadvertent deviations from such predefined distances (dx, dy) when in the expanded state may facilitate consistent and reliable functionality of electrodes (37).

In some procedures, it may be desirable to increase the area over which stresses are distributed across various portions of loops (100, 200, 200') to thereby reduce or eliminate the amounts of strain experienced by each loop (100, 200, 200'). For example, it may be desirable to increase the area over which stresses are distributed across the portions of loops (100, 200, 200') that are subjected to the highest amounts of stresses during normal use. In this regard, it will be appreciated that the retraction of end effector (14) relative to tubular sheath (13) and/or the direction of end effector (14) into tubular sheath (13) via the insertion tool may cause the highest amounts of stresses experienced by loops (100, 200, 200') at or near the respective distal segments (102D, 102E, 202D, 202'D), thereby resulting in relatively high strain points at or near the respective distal segments (102D, 102E, 202D, 202'D). Such high strain points may ultimately result in permanent deformation of the underlying frames (102, 202 and 202'). Increasing the area over which stresses are distributed across distal segments (102D, 102E, 202D, 202'D) of loops (100, 200, 200') may inhibit such permanent deformation from occurring, such that loops (100, 200, 200') may consistently and reliably expand from the collapsed state to the desired expanded state in which electrodes (37) may be spaced apart from each other by predefined distances, such as distances (dx, dy) described above. As mentioned above, avoiding inadvertent deviations from such predefined distances (dx, dy) when in the expanded state may facilitate consistent and reliable functionality of electrodes (37).

FIGS. 3-3B depict an example of an end effector (310) having such a configuration, and which may be incorporated into catheter (10) in place of end effector (14). End effector (310) may be similar to end effector (14) described above except as otherwise described below. In this regard, end effector (310) of this example includes first and second outer loop members (312a, 312b) and an inner loop member (314), each extending distally from a base member or shaft (316), which itself extends along longitudinal axis (L-L). Loop members (312a, 312b, 314) may also be referred to as paddles, loops, and/or electrode loop assemblies.

In the example shown, first and second outer loop members (312a, 312b) are substantially identical to each other and are disposed on either side of longitudinal axis (L-L), at least partially radially outwardly of third or central loop member (314). More particularly, outer loop members (312a, 312b) each include a corresponding outermost spine member (320a, 320b) and a corresponding innermost spine member (322a, 322b), while inner loop member (314) includes a pair of intermediate spine members (324a, 324b). Spine members (320a, 320b, 322a, 322b, 324a, 324b) may also be referred to as spines. In this regard, outermost spines (320a, 320b) are positioned radially outwardly of intermediate spines (324a, 324b) with respect to longitudinal axis (L-L), and innermost spines (322a, 322b) are positioned radially inwardly of intermediate spines (324a, 324b) with respect to longitudinal axis (L-L), at least within the frame of reference of FIG. 3. It will be appreciated that first outermost spine member (320a) is integral with first innermost spine member (322a) as part of first outer loop (312a) and that second outermost spine member (320b) is integral with second innermost spine member (322b) as part of second outer loop (312b). Similarly, first intermediate spine member (324a) is integral with second intermediate spine member (324b) as part of inner loop (314).

Loop members (312a, 312b, 314) also each include a corresponding plurality of sensing electrodes (330) secured to the respective spine members (320a, 320b, 322a, 322b, 324a, 324b). Electrodes (330) may be configured to provide electrophysiology (EP) mapping, such as to identify tissue regions that should be targeted for ablation. For example, electrodes (330) may monitor electrical signals emanating from conductive endocardial tissues to pinpoint the location of aberrant conductive tissue sites that are responsible for an arrhythmia. By way of example only, electrodes (330) may be configured and operable in accordance with at least some of the teachings of U.S. Pub. No. 2020/0345262, entitled "Mapping Grid with High Density Electrode Array," published November 5, 2020.

In the example shown, each loop member (312a, 312b, 314) includes a corresponding structural member (340a, 340b, 342). In this regard, each outer loop member (312a, 312b) has a corresponding elongated structural member (340a, 340b) extending continuously through the length of the respective outer loop member (312a, 312b) to and from base member (316), while inner loop member (314) has an elongated structural member (342) extending continuously through the length of inner loop member (314) to and from base member (316). Structural members (340a, 340b, 342) may also be referred to as loop structural members, spine frames, or frames. Structural members (340a, 340b, 342) can each include a biocompatible metal such as stainless steel, cobalt chromium, or nitinol.

As best shown in FIG. 3A, structural members (340a, 340b) of outer loops (312a, 312b) are each formed as a unitary structural framework defining respective pluralities of segments (351a, 351b, 352a, 352b, 353a, 353b, 354a, 354b, 355a, 355b, 356a, 356b, 357a, 357b, 358a, 358b, 359a, 359b, 360a, 360b, 361a, 361b) having varying widths (W1, W2, W3, W4, W5, W6); and structural member (342) of inner loop (314) is formed as a unitary structural framework defining a plurality of segments (371, 372, 373, 374, 375) having varying widths (W7, W8, W9).

More particularly, structural members (340a, 340b) of outer loops (312a, 312b) each include a respective first segment (351a, 351b) extending distally from base member (316), and having a first width (W1); a respective second segment (352a, 352b) extending distally and/or laterally inwardly from the respective first segment (351a, 351b), and having a second width (W2) less than the first width (W1) of first segments (351a, 351b); a respective third segment (353a, 353b) extending distally and/or laterally inwardly from the respective second segment (352a, 352b), and having a third width (W3) greater than the second width (W2) of second segments (352a, 352b); a respective fourth segment (354a, 354b) extending distally and/or laterally inwardly from the respective third segment (353a, 353b), and having a fourth width (W4) greater than the third width (W3) of third segments (353a, 353b); a respective fifth segment (355a, 355b) extending distally and/or laterally inwardly from the respective fourth segment (354a, 354b), and having a fifth width (W5) greater than the fourth width (W4) of fourth segments (354a, 354b); a respective sixth segment (356a, 356b) extending distally and/or laterally inwardly from the respective fifth segment (355a, 355b), and having a sixth width (W6) greater than the fifth width (W5) of fifth segments (355a, 355b); a respective seventh segment (357a, 357b) extending proximally and/or laterally inwardly from the respective sixth segment (356a, 356b), and having the same fifth width (W5) as fifth segments (355a, 355b); a respective eighth segment (358a, 358b) extending proximally and/or laterally inwardly from the respective seventh segment (357a, 357b), and having the same fourth width (W4) as fourth segments (354a, 354b); a respective ninth segment (359a, 359b) extending proximally and/or laterally inwardly from the respective eighth segment (358a, 358b), and having the same third width (W3) as third segments (353a, 353b); a respective tenth segment (360a, 360b) extending proximally and/or laterally inwardly from the respective ninth segment (359a, 359b) (and curving laterally outwardly in the example shown), and having the same second width (W2) as second segments (352a, 352b); and a respective eleventh segment (361a, 361b) extending proximally from the respective tenth segment (360a, 360b) to base member (316), and having the same first width (W1) as first segments (351a, 351b). As discussed in greater detail below, the transitions between adjacent widths, such as the transition from the first width (W1) to the second width (W2), may be gradual (e.g., tapered).

Structural member (342) of inner loop (314) includes a first segment (371) extending distally from base member (316), and having a seventh width (W7); a second segment (372) extending distally and/or laterally inwardly from first segment (371), and having an eighth width (W8) less than the seventh width (W7) of first segment (371); a third segment (373) extending laterally across the longitudinal axis (L-L) from second segment (372), and having a ninth width (W9) greater than the eighth width (W8) of second segment (372); a fourth segment (374) extending proximally and/or laterally outwardly from third segment (373), and having the same eighth width (W8) as second segment (372); and a fifth segment (375) extending proximally from fourth segment (374) to base member (316), and having the same seventh width (W7) as first segment (371). In some versions, the seventh width (W7) may be the same as the first width (W1); the eighth width (W8) may be the same as the second width (W2); and/or the ninth width (W9) may be the same as the sixth width (W6).

The first width (W1) may be about 0.01 inch, for example; the second width (W2) may be about 0.005 inch, for example; the third width (W3) may be about 0.00525 inch, for example; the fourth width (W4) may be about 0.00550 inch, for example; the fifth width (W5) may be about 0.00575 inch, for example; and/or the sixth width (W6) may be about 0.006 inch, for example. Thus, the sixth width (W6) may be about 20% greater than the second width (W2) in some versions.

As shown, third, fourth, fifth, sixth, seventh, eighth, and ninth segments (353a, 353b, 354a, 354b, 355a, 355b, 356a, 356b, 357a, 357b, 358a, 358b, 359a, 359b) are positioned at distal portions of the respective outer loops (312a, 312b), at which the highest amounts of stress may be experienced by outer loops (312a, 312b) during normal use. In some versions, sixth segments (356a, 356b) may be positioned at or near the distal-most portions of the respective outer loops (312a, 312b). Due to the increased magnitudes of the third, fourth, fifth, and sixth widths (W3, W4, W5, W6) relative to the second width (W2), third, fourth, fifth, sixth, seventh, eighth, and ninth segments (353a, 353b, 354a, 354b, 355a, 355b, 356a, 356b, 357a, 357b, 358a, 358b, 359a, 359b) may provide an increased area over which the stresses are distributed across the distal portion of the respective outer loop (312a, 312b) (e.g., at least by comparison to scenarios in which the third, fourth, fifth, and/or sixth widths (W3, W4, W5, W6) are substantially equal to or less than the second width (W2)). In this manner, third, fourth, fifth, sixth, seventh, eighth, and ninth segments (353a, 353b, 354a, 354b, 355a, 355b, 356a, 356b, 357a, 357b, 358a, 358b, 359a, 359b) of each outer loop (312a, 312b) may collectively define a corresponding stress distribution node (368a, 368b) of the respective outer loop (312a, 312b). In some versions, stress distribution node (368a, 368b) of each outer loop (312a, 312b) may be defined by more or less than third, fourth, fifth, sixth, seventh, eighth, and ninth segments (353a, 353b, 354a, 354b, 355a, 355b, 356a, 356b, 357a, 357b, 358a, 358b, 359a, 359b). For example, stress distribution node (368a, 368b) of each outer loop (312a, 312b) may be defined by only the respective sixth segment (356a, 356b), with third, fourth, fifth, seventh, eighth, and ninth segments (353a, 353b, 354a, 354b, 355a, 355b, 357a, 357b, 358a, 358b, 359a, 359b) being omitted such that sixth segments (356a, 356b) are interposed directly between the respective second and tenth segments (352a, 352b, 360a, 360b).

Likewise, third segment (373) is positioned at a distal portion of inner loop (314), at which the highest amounts of stress may be experienced by inner loop (314) during normal use. Third segment (373) is positioned at the distal-most portion of inner loop (314). Due to the increased magnitude of the ninth width (W9) relative to the eighth width (W8), third segment (373) may provide an increased area over which the stresses are distributed across the distal portion of inner loop (314) (e.g., at least by comparison to scenarios in which the ninth width (W9) is substantially equal to or less than the eighth width (W8)). In this manner, third segment (373) of inner loop (314) may define a corresponding stress distribution node (378) of inner loop (314).

In some versions, first segments (351a, 351b) of outer loops (312a, 312b) may have a first thickness (not shown), and eleventh segments (361a, 361b) of outer loops (312a, 312b) may have the same first thickness as first segments (351a, 351b). The first thickness may be about 0.01 inch, for example. In addition, or alternatively, second segments (352a, 352b) of outer loops (312a, 312b) may have a second thickness (not shown) less than the first thickness, and any one or more of third segments (353a, 353b), fourth segments (354a, 354b), fifth segments (355a, 355b), sixth segments (356a, 356b), seventh segments (357a, 357b), eighth segments (358a, 358b), ninth segments (359a, 359b), and/or tenth segments (360a, 360b) of outer loops (312a, 312b) may have the same second thickness as second segments (352a, 352b). The second thickness may be about 0.005 inch, for example. First segment (371) and fifth segment (375) of inner loop (314) may have the same first thickness as first segments (351a, 351b) and eleventh segments (361a, 361b) of outer loops (312a, 312b); and second, third, and fourth segments (372, 373, 374) of inner loop (314) may have the same second thickness as second, third, fourth, fifth, sixth, seventh, eight, ninth, and/or tenth segments (352a, 352b, 353a, 353b, 354a, 354b, 355a, 355b, 356a, 356b, 357a, 357b, 358a, 358b, 359a, 359b, 360a, 360b) of outer loops (312a, 312b).

In the example shown, the first width (W1) of first segments (351a, 351b) and eleventh segments (361a, 361b) is substantially equal to the first thickness of first segments (351a, 351b) and eleventh segments (361a, 361b), such that first segments (351a, 351b) and eleventh segments (361a, 361b) each have a generally square cross-sectional shape; the second width (W2) of second segments (352a, 352b) and tenth segments (360a, 360b) is substantially equal to the second thickness of second segments (352a, 352b) and tenth segments (360a, 360b), such that second segments (352a, 352b) and tenth segments (360a, 360b) each have a generally square cross-sectional shape; the third width (W3) of third segments (353a, 353b) and ninth segments (359a, 359b) is substantially greater than the second thickness of third segments (353a, 353b) and ninth segments (359a, 359b), such that third segments (353a, 353b) and ninth segments (359a, 359b) each have a generally rectangular cross-sectional shape; the fourth width (W4) of fourth segments (354a, 354b) and eighth segments (358a, 358b) is substantially greater than the second thickness of fourth segments (354a, 354b) and eighth segments (358a, 358b), such that fourth segments (354a, 354b) and eighth segments (358a, 358b) each have a generally rectangular cross-sectional shape; the fifth width (W5) of fifth segments (355a, 355b) and seventh segments (357a, 357b) is substantially greater than the second thickness of fifth segments (355a, 355b) and seventh segments (357a, 357b), such that fifth segments (355a, 355b) and seventh segments (357a, 357b) each have a generally rectangular cross-sectional shape; and the sixth width (W6) of sixth segments (356a, 356b) is greater than the second thickness of sixth segments (356a, 356b), such that sixth segments (356a, 356b) each have a generally rectangular cross-sectional shape.

As shown, each transition between adjacent pairs of segments (351a, 351b, 352a, 352b, 353a, 353b, 354a, 354b, 355a, 355b, 356a, 356b, 357a, 357b, 358a, 358b, 359a, 359b, 360a, 360b, 361a, 361b, 371, 372, 373, 374, 375) may have a stepped configuration. In some other versions, the transitions between any one or more adjacent pairs of segments (351a, 351b, 352a, 352b, 353a, 353b, 354a, 354b, 355a, 355b, 356a, 356b, 357a, 357b, 358a, 358b, 359a, 359b, 360a, 360b, 361a, 361b, 371, 372, 373, 374, 375) may have continuously tapered configurations. For example, structural members (340a, 340b) may each taper outwardly (relative to a central axis of the respective structural member (340a, 340b)) from the respective second segment (352a, 352b) to the respective third segment (353a, 353b); from the respective third segment (353a, 353b) to the respective fourth segment (354a, 354b); from the respective fourth segment (354a, 354b) to the respective fifth segment (355a, 355b); and/or from the respective fifth segment (355a, 355b) to the respective sixth segment (356a, 356b). In addition, or alternatively, structural members (340a, 340b) may each taper inwardly (relative to a central axis of the respective structural member (340a, 340b)) from the respective sixth segment (356a, 356b) to the respective seventh segment (357a, 357b); from the respective seventh segment (357a, 357b) to the respective eighth segment (358a, 358b); from the respective eighth segment (358a, 358b) to the respective ninth segment (359a, 359b); and/or from the respective ninth segment (359a, 359b) to the respective tenth segment (360a, 360b). In some versions, structural members (340a, 340b) may each taper outwardly (relative to a central axis of the respective structural member (340a, 340b)) continuously from the respective second segment (352a, 352b) to the respective sixth segment (356a, 356b), and/or may each taper inwardly (relative to a central axis of the respective structural member (340a, 340b)) continuously from the respective sixth segment (356a, 356b) to the respective tenth segment (360a, 360b).

In the example shown, the third, fourth, fifth, sixth, and ninth widths (W3, W4, W5, W6, W9) are each defined by the respective structural member (340a, 340b, 342) itself. In some other versions, one or more sleeves (not shown) may be applied over structural members (340a, 340b, 342) to define any one or more of the third, fourth, fifth, sixth, and/or ninth widths (W3, W4, W5, W6, W9). In this manner, such sleeves may contribute to defining the respective stress distribution nodes (368a, 368b, 378). It will be appreciated that such sleeves may comprise the same material as that of the respective structural member (340a, 340b, 342) and/or a different material from that of the respective structural member (340a, 340b, 342).

While stress distribution nodes (368a, 368b) of outer loops (312a, 312b) in the present example are defined by third, fourth, fifth, sixth, seventh, eighth, and ninth segments (353a, 353b, 354a, 354b, 355a, 355b, 356a, 356b, 357a, 357b, 358a, 358b, 359a, 359b) via the increased magnitudes of the third, fourth, fifth, and sixth widths (W3, W4, W5, W6) relative to the second width (W2), it will be appreciated that stress distribution nodes (368a, 368b) of outer loops (312a, 312b) may additionally or alternatively be defined by increased magnitudes of the thickness of third, fourth, fifth, sixth, seventh, eighth, and ninth segments (353a, 353b, 354a, 354b, 355a, 355b, 356a, 356b, 357a, 357b, 358a, 358b, 359a, 359b) relative to that of second and tenth segments (352a, 352b, 360a, 360b). For example, rather than each having the same second thickness as second and tenth segments (352a, 352b, 360a, 360b), third, fourth, fifth, sixth, seventh, eighth, and ninth segments (353a, 353b, 354a, 354b, 355a, 355b, 356a, 356b, 357a, 357b, 358a, 358b, 359a, 359b) may have respective thicknesses that are substantially greater than the second thickness, such as third, fourth, fifth, and sixth thicknesses with magnitudes similar to those described above for the third, fourth, fifth, and sixth widths (W3, W4, W5, W6), respectively.

Each of the stress distribution nodes (368a, 368b) may have its thickest portion (i.e., largest cross-sectional area) located at a respective distance (RB1, RB2) from a transverse axis (T-T) (which is perpendicular to the longitudinal axis (L-L)). The transverse axis (T-T) intersects structural member (340a) at a location where curved segment (352a) and linear segment (351a) comes together at interface (351c). The distance (RB1) may be from about 2 mm to about 4 mm, such as about 3mm. The distance (RB2) may have the same range as distance (RB1).

Similarly, while stress distribution node (378) of inner loop (314) in the present example is defined by third segment (373) via the increased magnitude of the ninth width (W9) relative to the eighth width (W8), it will be appreciated that stress distribution node (378) of inner loop (314) may additionally or alternatively be defined by an increased magnitude of the thickness of third segment (373) relative to that of second and fourth segments (372, 374). For example, rather than each having the same second thickness as second and fourth segments (372, 374), third segment (373) may have a thickness that is substantially greater than the second thickness, such as a thickness with a magnitude similar to that described above for the ninth width (W9).

As shown in FIG. 3B, each loop member (312a, 312b, 314) may further include a corresponding cover (380a, 380b, 382), with the corresponding structural member (340a, 340b, 342) (including the corresponding stress distribution node (368a, 368b, 370) underlying the corresponding cover (380a, 380b, 382) and with the corresponding plurality of electrodes (330) disposed on an outer surface of the corresponding cover (380a, 380b, 382). Covers (380a, 380b, 382) may each be electrically insulative, and/or may each include a polymeric material such as polyurethane, for example. By way of example only, each cover (380a, 380b, 382) may be provided as a coating, as an overmold, or in some other suitable fashion.

### Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein.

Having shown and described various versions of the present invention, further adaptations of the methods and systems described herein may be accomplished by appropriate modifications by one skilled in the art. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention is defined by the following claims and is understood not to be limited to the details of structure and operation shown and described in the specification and drawings.

## Claims

1. An end effector (310) of a catheter (10), the end effector comprising:
(a) three loop members (312a, 312b, 314), wherein the three loop members include a pair of outer loop members (312a, 312b) and an inner loop member (314) positioned radially inwardly of the pair of outer loop members relative to the longitudinal axis, each loop member of the three loop members including an elongate structural member, each elongate structural member including:
(i) at least one proximal segment having a first cross dimension, and
(ii) at least one distal segment positioned at a distal portion of the elongate structural member and positioned distally of the at least one proximal segment and having a second cross dimension greater than the first cross dimension; and
(b) a plurality of electrodes affixed to the plurality of loop members, and
**characterised in that**
the at least one distal segment of the inner loop member is positioned at the distal-most point of the elongate structural member of the inner loop member.

2. The end effector of claim 1, the second cross dimension being about 20% greater than the first cross dimension.

3. A catheter for electrophysiology applications, comprising:
(a) a shaft extending along a longitudinal axis to a distal end; and
(b) the end effector of claim 1 or claim 2.

4. The catheter of claim 3, the first cross dimension including a first width, the second cross dimension including a second width, or the first cross dimension including a first thickness, the second cross dimension including a second thickness.

5. The catheter of claim 3, the elongate structural member of each loop member transitioning from the at least one proximal segment to the at least one distal segment via a stepped configuration, or via a tapered configuration.

6. The catheter of claim 3, each loop member of the plurality of loop members including a cover disposed about the corresponding elongate structural member.

## Patentansprüche

1. Endeffektor (310) eines Katheters (10), der Endeffektor umfassend:
(a) drei Schlaufenelemente (312a, 312b, 314), wobei die drei Schlaufenelemente ein Paar von äußeren Schlaufenelementen (312a, 312b) und ein inneres Schlaufenelement (314) einschließen, das relativ zu der Längsachse radial einwärts von dem Paar von äußeren Schlaufenelementen positioniert ist, wobei jedes Schlaufenelement der drei Schlaufenelemente ein längliches Strukturelement einschließt, wobei jedes längliche Strukturelement einschließt:
(i) mindestens ein proximales Segment, das eine erste Querabmessung aufweist, und
(ii) mindestens ein distales Segment, das an einem distalen Abschnitt des länglichen Strukturelements positioniert ist und distal von dem mindestens einen proximalen Segment positioniert ist und eine zweite Querabmessung aufweist, die größer als die erste Querabmessung ist; und
(b) eine Vielzahl von Elektroden, die an der Vielzahl von Schlaufenelementen befestigt sind, und
**dadurch gekennzeichnet, dass**
das mindestens eine distale Segment des inneren Schlaufenelements an dem distalsten Punkt des länglichen Strukturelements des inneren Schlaufenelements positioniert ist.

2. Endeffektor nach Anspruch 1, wobei die zweite Querabmessung etwa 20 % größer als die erste Querabmessung ist.

3. Katheter für elektrophysiologische Anwendungen, umfassend:
(a) einen Schaft, der sich entlang einer Längsachse zu einem distalen Ende erstreckt; und
(b) den Endeffektor nach Anspruch 1 oder 2.

4. Katheter nach Anspruch 3, wobei die erste Querabmessung eine erste Breite einschließt, die zweite Querabmessung eine zweite Breite einschließt, oder die erste Querabmessung eine erste Dicke einschließt, die zweite Querabmessung eine zweite Dicke einschließt.

5. Katheter nach Anspruch 3, wobei das längliche Strukturelement jedes Schlaufenelements von dem mindestens einen proximalen Segment in das mindestens eine distale Segment über eine gestufte Konfiguration oder über eine verjüngte Konfiguration übergeht.

6. Katheter nach Anspruch 3, wobei jedes Schlaufenelement der Vielzahl von Schlaufenelementen eine Abdeckung einschließt, die um das entsprechende längliche Strukturelement herum angeordnet ist.

## Revendications

1. Effecteur terminal (310) d'un cathéter (10), l'effecteur terminal comprenant :
a) trois éléments boucles (312a, 312b, 314), dans lequel les trois éléments boucles comportent une paire d'éléments boucles externes (312a, 312b) et un élément boucle interne (314) positionné de manière radiale vers l'intérieur de la paire d'éléments boucles externes par rapport à l'axe longitudinal, chaque élément boucle des trois éléments boucles comportant un élément structurel allongé, chaque élément structurel allongé comportant :
(i) au moins un segment proximal ayant une première dimension transversale,
(ii) au moins un segment distal positionné au niveau d'une partie distale de l'élément structurel allongé et positionné de manière distale par rapport à l'au moins un segment proximal et ayant une seconde dimension transversale supérieure à la première dimension transversale ; et
b) une pluralité d'électrodes fixée à la pluralité d'éléments boucles ; et
**caractérisé en ce que**
l'au moins un segment distal de l'élément boucle interne est positionné au niveau du point le plus distal de l'élément structurel allongé de l'élément boucle interne.

2. Effecteur terminal selon la revendication 1, la seconde dimension transversale étant environ 20 % supérieure à la première dimension transversale.

3. Cathéter destiné à des applications électrophysiologiques, comprenant :
(a) un arbre s'étendant le long d'un axe longitudinal jusqu'à une extrémité distale ; et
(b) l'effecteur terminal selon la revendication 1 ou la revendication 2.

4. Cathéter selon la revendication 3, la première dimension transversale comportant une première largeur, la seconde dimension transversale comportant une seconde largeur, ou la première dimension transversale comportant une première épaisseur, la seconde dimension transversale comportant une seconde épaisseur.

5. Cathéter selon la revendication 3, l'élément structurel allongé de chaque élément boucle passant de l'au moins un segment proximal à l'au moins un segment distal par l'intermédiaire d'une configuration en gradins ou par l'intermédiaire d'une configuration conique.

6. Cathéter selon la revendication 3, chaque élément boucle de la pluralité d'éléments boucles comportant un couvercle disposé autour de l'élément structurel allongé correspondant.
